# EUROPÄISCHE PATENTSCHRIFT

(11) **EP 3 266 484 B1**
(45) Veröffentlichungstag und Bekanntmachung des Hinweises auf die Patenterteilung: **21.11.2018**
(21) Anmeldenummer: 17000810.6
(22) Anmeldetag: 27.08.2013
(51) Int. Cl.: A61M 16/16, A61M 16/10

(54) **VORRICHTUNG ZUR ANFEUCHTUNG VON ATEMGAS**
DEVICE FOR THE HUMIDIFICATION OF RESPIRATORY GAS
DISPOSITIF D'HUMIDIFICATION DE GAZ RESPIRATOIRE

(30) Priorität: 28.08.2012 DE 102012016972
(43) Veröffentlichungstag der Anmeldung: 10.01.2018
(62) Teilanmeldung aus: 13004229.4
(73) Patentinhaber: Löwenstein Medical Technology S.A., 2557 Luxembourg (LU)
(72) Erfinder: Feldhahn, Karl-Andres, 22761 Hamburg (DE); Hein, Stefan, 22529 Hamburg (DE); König, Karl-Heinz, 22850 Norderstedt (DE); Sepke, Heiko, 22952 Lütjensee (DE); Gardein, Joachim, 38430 Icod de los Vinos Tenerife / Islas Canarias (ES)
(74) Vertreter: Marx, Thomas

(56) Entgegenhaltungen:
- US-A1- 2006 237 005
- US-A1- 2011 155 132

## Beschreibung

Eine typische Verwendung von Atemluftanfeuchtern erfolgt in Zusammenhang mit Atemluftversorgungen, die im Rahmen einer CPAP-Therapie (Continious-Positive-Airway-Pressure), Bilevel-, APAP-oder Heimbeatmung eingesetzt werden. Sie vermeiden ein Austrocknen der Atemwege insbesondere bei längeren Beatmungsphasen. Der Atemluftanfeuchter weist in der Regel einen befüllbaren Wassertank und ein Heizelement auf.

Aufgrund der einzuhaltenden hygienischen Anforderungen an einen Atemluftanfeuchter, muss dieser leicht zu demontieren bzw. zu montieren und zu reinigen sein.

Bei der DE10049869A1 wird Atemluftanfeuchter zwischen Patientenschlauchsystem und Beatmungsgerät angeordnet. Dadurch muss zur Reinigung und auch zur Befüllung des Anfeuchters das Patientenschlauchsystem demontiert werden, was einen erhöhten Aufwand für die Pflege und Reinigung bedeutet. Auch bei der DE102006034028A1 muss zur Reinigung und auch zur Befüllung des Anfeuchters das Patientenschlauchsystem demontiert werden. Aus der US 2006/237005 A1 ist ein Befeuchter mit einem ersten Befeuchtungsteil (Abdeckung) und einem zweiten Befeuchtungsteil (Basis), der mit dem ersten Befeuchtungsteil verbindbar ist, bekannt. Der Befeuchter umfasst eine Dichtung, die zwischen der oberen Abdeckung und der Basis ausgebildet ist.

Alle bekannten Anfeuchter sind vielteilig aufgebaut. Die grosse Anzahl an trennbaren Einzelbestandteilen erschwert die Montage und Demontage und somit Reinigung der Anfeuchter.

Die Erfindung ist in den angehängten Ansprüchen definiert. Ziel der Erfindung ist es einen Atemluftanfeuchter bereitzustellen, der an die Bedürfnisse des Anwenders angepasst ist.

In dem Ausführungsbeispiel ist der Atemluftanfeuchter als modulares Funktionselement ausgeführt, welches bei Bedarf mit nur wenigen Handgriffen an das Beatmungsgerät konnektiert und so den individuellen Bedürfnissen des Anwenders angepasst wird.

Der Atemluftanfeuchter ist zur Verwendung mit einem Beatmungsgerät vorgesehen und besteht aus einer gegenüber dem Stand der Technik reduzierten Anzahl an Teilen, nämlich einem Gehäuseunterteil mit einem direkten oder indirekten Heizelement, einem Gehäuseoberteil und einem elastomeren Mittelteil, welches einstückig ist und eine Dichtauflage, die zumindest teilweise als Dichtfläche ausgestaltet sein kann, zum Gehäuseunterteil aufweist.

Der erfindungsgemässe Anfeuchter besteht somit bevorzugt aus sechs manuell trennbaren Einzelbauteilen.

Der erfindungsgemässe Anfeuchter besteht somit besonders bevorzugt aus fünf manuell trennbaren Einzelbauteilen.

Der erfindungsgemässe Anfeuchter besteht somit besonders bevorzugt aus vier manuell trennbaren Einzelbauteilen.

Dies wird insbesondere erreicht durch eine Funktionsintegration im Bereich des Mittelteiles. Das Mittelteil ist einstückig ausgebildet und dient der Strömungsführung, der Schalldämmung, der Befüllung mit Wasser und der Wasserführung, der Abdichtung zwischen Ober- und Unterteil, sowie der Abdichtung zum Beatmungsgerät hin. Der Atemluftanfeuchter ist so aufgebaut, dass das Mittelteil einstückig ausgebildet ist und Elemente zur Strömungsführung und/oder Strömungskanäle und Dichtelemente zur pneumatischen Abdichtung aufweist. Der Atemluftanfeuchter ist zur Verwendung mit einem Beatmungsgerät vorgesehen und besteht aus einem Gehäuseunterteil mit direkten oder indirekten Heizelement, einem Gehäuseoberteil und einem elastomeren Mittelteil, welches eine Dichtauflage, die zumindest teilweise als Dichtfläche ausgestaltet sein kann, zum Gehäuseunterteil aufweist. Der Atemluftanfeuchter weist eine kompakte Gehäusekonstruktion auf, die dem Konturverlauf des Beatmungsgerätes angepasst ist. Der Atemluftanfeuchter ist auch dadurch gekennzeichnet, dass im Gehäuseoberteil als Öffnungen ein Lufteinlass und ein Luftauslass zur luftführenden Kopplung mit dem Beatmungsgerät vorgesehen sind. Der Atemluftanfeuchter ist auch dadurch gekennzeichnet, dass der Lufteinlass und/oder der Luftauslass auch als Einfüllöffnungen für das Wasser dienen. Der Atemluftanfeuchter ist auch dadurch gekennzeichnet, dass räumlich benachbart zu der Öffnung zumindest eine erhöhte Kante als Überlaufschutz angeordnet ist. Der Atemluftanfeuchter ist auch dadurch gekennzeichnet, dass die Fläche im Bereich neben der Öffnung leicht trichterförmig ausgeführt ist, um das einzufüllende Wasser in Richtung der Öffnung zu führen. Der Atemluftanfeuchter ist auch dadurch gekennzeichnet, dass im Gehäuseoberteil zumindest ein Griff bereitgestellt wird. Der Atemluftanfeuchter ist auch dadurch gekennzeichnet, dass im Gehäuseunterteil zwei von außen sichtbare Füllstandanzeigen angeordnet sind, die eine maximale Füllhöhe anzeigen. Der Atemluftanfeuchter ist auch dadurch gekennzeichnet, dass durch die gekrümmt angeordneten Luftkanäle des Mittelteiles zudem ein Zurück- bzw. Auslaufen der Flüssigkeit aus dem Anfeuchter verhindern wird. Der Atemluftanfeuchter ist auch dadurch gekennzeichnet, dass eine einhändige Konnektierung des Anfeuchters an das Beatmungsgerät durch korrespondierende Abmessungen im Bereich der Kontaktstelle und einen Schnappmechanismus zur Befestigung erreicht wird. Der Atemluftanfeuchter ist auch dadurch gekennzeichnet, dass der Anfeuchter in einem Seitenbereich an das Beatmungsgerät konnektiert wird und der Luftweg im Bypass durch den Anfeuchter und wieder zurück in das Beatmungsgerät geführt wird, wobei der Schlauchanschluss am Beatmungsgerät verbleibt. Der Atemluftanfeuchter ist auch dadurch gekennzeichnet, dass das Mittelteil zumindest ein Dichtelement zur Abdichtung zum Beatmungsgerät hin aufweist. Der Atemluftanfeuchter ist auch dadurch gekennzeichnet, dass das Mittelteil aus mindestens 2 Elastomeren verschiedener Härtegrade (Shorehärten) besteht. Der Atemluftanfeuchter ist auch dadurch gekennzeichnet, dass das elastomere Mittelteil in einer 2K- Technik hergestellt wird, Der Atemluftanfeuchter ist auch dadurch gekennzeichnet, dass die strömungsführenden- Elemente des elastomeren Mittelteils eine höhere Shorehärte und die Dicht(-auflage)-elemente des elastomeren Mittelteils eine niedrigere Shorehärte aufweisen, Der Atemluftanfeuchter ist auch dadurch gekennzeichnet, dass die strömungsführenden Elemente des elastomeren Mittelteils eine Shorehörte zwischen 40 und 100 Shore A und die Dicht(-auflage) -elemente des elastomeren Mittelteils eine Shorehärte zwischen 5 und 70 Shore A aufweisen.

Der Atemluftanfeuchter ist auch dadurch gekennzeichnet, dass die Dicht(-auflage)-elemente des elastomeren Mittelteils den von Mittelteil und Gehäuseunterteil gemeinsam gebildeten Wasseraufnahmeraum gegenüber der Umgebung abdichten.

Der Atemluftanfeuchter ist auch dadurch gekennzeichnet, dass das Mittelteil Elemente zur Strömungsführung und/oder Strömungskanäle aufweist.

Der Atemluftanfeuchter ist auch dadurch gekennzeichnet, dass die Elemente zur Strömungsführung und/oder Strömungskanäle mindestens eine Umlenkung der Luft von horizontal nach vertikal und/oder eine Umlenkung von vertikal in eine horizontale Strömungsrichtung zum Anfeuchterausgang hin bewirken.

Der Atemluftanfeuchter ist auch dadurch gekennzeichnet, dass die Strömungsführungselemente des Mittelteils mindestens eine Umlenkung aus einer horizontalen Strömungsrichtung (vom Beatmungsgerät kommend) in eine vertikale Strömungsrichtung (auf das Wasser zu) bewirken.

Der Atemluftanfeuchter ist auch dadurch gekennzeichnet, dass die Strömungsführungselemente des elastomeren Mittelteils mindestens eine Umlenkung aus einer vertikalen Strömungsrichtung (vom Wasser weg) in eine horizontale Strömungsrichtung (zum Beatmungsgerät bzw. zum Geräteausgang hin) bewirken.

Der Atemluftanfeuchter ist auch dadurch gekennzeichnet, dass die Strömungsführungselemte des Mittelteils horizontale Umlenkungen in der Luftströmung oberhalb der Wasseroberfläche bewirken und durch diese Umlenkungen bei gegebenem Anfeuchterraum einen langen Strömungsweg der Luft über dem Wasser zur Aufnahme von Wassermolekülen in der Luft erzwingen.

Der Atemluftanfeuchter ist auch dadurch gekennzeichnet, dass die Elemente zur Strömungsführung und/oder Strömungskanäle eine Aufteilung der Luftströmung in mindestens zwei Teilströme oberhalb der Wasseroberfläche bewirken und die Teilströme vor dem Luftauslass wieder vereinigt werden.

Der Atemluftanfeuchter ist auch dadurch gekennzeichnet, dass das Mittelteil des Atemluftanfeuchters zum Einen der Luftführung durch den Anfeuchter, zum Anderen der Abdichtung zwischen Ober- und unterteil und auch der Wasserführung beim Befüllvorgang dient.

Der Atemluftanfeuchter ist auch dadurch gekennzeichnet, dass der Lufteinlass und der Luftauslass auch als Einfüllöffnungen für das Wasser dienen.

Der Atemluftanfeuchter ist auch dadurch gekennzeichnet, dass im Gehäuseoberteil als Öffnungen ein Lufteinlass und ein Luftauslass zur luftführenden Kopplung mit dem Beatmungsgerät vorgesehen sind.

Der Atemluftanfeuchter ist auch dadurch gekennzeichnet, dass das elastomere Mittelteil rohrförmige Anschlussstellen bereitstellt die mit den Öffnungen im Gehäuseoberteil gemeinsam jeweils einen Lufteinlass und einen Luftauslass bilden.

Der Atemluftanfeuchter ist auch dadurch gekennzeichnet, dass die rohrförmigen Anschlussstellen im elastomeren Mittelteil, im Kopplungsbereich zum Beatmungsgerät Dichtelemente zur Abdichtung zum Beatmungsgerät hin aufweisen.

Der Atemluftanfeuchter ist auch dadurch gekennzeichnet, dass die Dichtelemente an den rohrförmigen Anschlussstellen eine Shorehärte zwischen 5 und 70 Shore A aufweisen.

Der Atemluftanfeuchter ist auch dadurch gekennzeichnet, dass Lufteinlass und Luftauslass benachbart angeordnet sind und eine Befüllung mit Wasser (ohne Fehlbedienungsrisiko) wahlweise durch eine der beiden Öffnungen vorgenommen werden kann.

Der Atemluftanfeuchter ist auch dadurch gekennzeichnet, dass
a) bei Befüllung des Anfeuchters über eine der Öffnungen der Wasserstand im Wasseraufnahmeraum bis zum oberen Rand des Kanals ansteigt, und dann die - unter dem Druck (F_{L}) stehende - Luft im Luftraum des Anfeuchters nicht weiter entweichen kann und
b) bei weiterer Befüllung und weiter steigendem und den Druck Fw ausübenden Wasserstand im Kanal die Luft unter dem ebenfalls steigenden Druck F_{L} komprimiert wird und kein weiteres Wasser in den Wasseraufnahmeraum fließen kann sondern dieses über die Öffnung nach außen abfließt.

Der Atemluftanfeuchter ist auch dadurch gekennzeichnet, dass Luftkanäle im Mittelteil beim Übergang zwischen vertikalen und horizontalen Abschnitten geometrische Überhöhungen enthalten, um während des Betriebes oder bei Transport und Verkippen ein Eindringen von Wasser in die horizontalen Strömungsabschnitte (zurück in das Gerät oder hin zum Geräteausgang) zumindest zu erschweren.

Der Atemluftanfeuchter ist auch dadurch gekennzeichnet, dass die vertikalen Luftkanäle im Bereich der Mitte des Anfeuchters angeordnet sind um während des Betriebes oder bei Transport und bei Verkippen ein Eindringen von Wasser in die horizontalen Strömungsabschnitte (zurück in das Gerät oder hin zum Geräteausgang) zumindest zu erschweren.

Der Atemluftanfeuchter ist auch dadurch gekennzeichnet, dass das Gehäuseoberteil im Wesentlichen der mechanischen Abstützung des elastomeren Mittelteils sowie der mechanischen Verriegelung mit dem Unterteil dient, wobei das Mittelteil zwischen Oberteil und Unterteil eingespannt wird.

Der Atemluftanfeuchter ist auch dadurch gekennzeichnet, dass zum Zusammenbau zunächst in einem einfachen Montageschritt das elastomere Mittelteil in das Oberteil eingesetzt wird woraufhin in einem zweiten ebenfalls einfachen Montageschritt die Einheit aus Gehäuseoberteil und elastomeren Mittelteil mit dem Gehäuseunterteil einrastend zusammengesetzt wird.

Der Atemluftanfeuchter ist auch dadurch gekennzeichnet, dass das Einrasten durch Rasthaken im Unterteil und entsprechende Öffnungen für die Rasthaken im Oberteil vorgesehen sind.

Der Atemluftanfeuchter ist auch dadurch gekennzeichnet, dass im Gehäuseunterteil Führungsaufnahmen zur Justierung des Gehäuseoberteiles angeordnet sind, in die entsprechende Führungsstege des Gehäuseoberteiles greifen die einen falschen Zusammenbau der Bauteile verhindern.

Der Atemluftanfeuchter ist auch dadurch gekennzeichnet, dass die Führungsstege und -aufnahmen asymmetrisch an Gehäuseunterteil und Oberteil angeordnet sind um eine definierte Orientierung bei der Montage vorzugeben.

Der Atemluftanfeuchter ist auch dadurch gekennzeichnet, dass die Führungsaufnahmen jeweils eine trichterförmige Öffnung aufweisen die zum Einführen der Führungsstege in die Führungsaufnahmen dienen.

Der Atemluftanfeuchter ist auch dadurch gekennzeichnet, dass zum Lösen der Verbindung zwischen Oberteil und Unterteil eine Grifffläche im Bereich unter einen Schenkel des Oberteiles vorgesehen ist und dass durch eine leichte Zugbewegung die Rastverbindung gelöst werden kann.

Der Atemluftanfeuchter ist auch dadurch gekennzeichnet, dass im Gehäuseoberteil zumindest ein Griff bereitgestellt wird,

Der Atemluftanfeuchter ist auch dadurch gekennzeichnet, dass im Gehäuseunterteil zwei von außen sichtbare Füllstandanzeigen angeordnet sind, die eine Füllhöhe anzeigen.

Der Atemluftanfeuchter ist auch dadurch gekennzeichnet, dass im Gehäuseunterteil mindestens zwei von außen sichtbaren Füllstandanzeigen zum Ablesen bei verschiedenen räumlichen Winkellagen (z.B. während des Befüllens und in der normalen horizontalen Gebrauchsanordnung) angeordnet sind, die eine maximale Füllhöhe anzeigen.

Der Atemluftanfeuchter ist auch dadurch gekennzeichnet, dass eine einhändige Konnektierung des Anfeuchters an das Beatmungsgerät durch korrespondierende Abmessungen im Bereich der Kontaktstelle und einen Schnappmechanismus zur Befestigung erreicht wird.

Der Atemluftanfeuchter ist auch dadurch gekennzeichnet, dass der Anfeuchter in einem Seitenbereich an das Beatmungsgerät konnektiert wird und der Luftweg durch den Anfeuchter und wieder zurück in das Beatmungsgerät geführt wird, wobei der Schlauchanschluss am Beatmungsgerät verbleibt.

Mittelteil für einen Atemluftanfeuchter ist dadurch gekennzeichnet, dass das Mitteilteil Elemente zur Strömungsführung und/oder Strömungskanäle aufweist, die aufgrund ihrer räumlichen Gestalt bei einem werkzeugbasierten Herstellungsprozesses elastische Nachgiebigkeit des Mittelteiles bei der Entformung voraussetzen.

Werkzeuggebundenes Verfahren zur Herstellung eines elastomeren Mittelteils für einen Atemluftanfeuchter dadurch gekennzeichnet, dass in dem Mittelteil zumindest ein in Richtung der Öffnung des Werkzeugs realisierter Hinterschnitt unter Beanspruchung der Nachgiebigkeit des elastomeren Werkstoffs mindestens bereichsweise zwangsentformt wird.

Werkzeuggebundenes Verfahren zur Herstellung eines elastomeren Mittelteils für einen Atemluftanfeuchters dadurch gekennzeichnet, dass das Mittelteil strömungsführende Elemente zur Realisierung von Umlenkungen der Strömung innerhalb des Atemluftanfeuchters bewirken wobei in dem Mittelteil in Richtung der Öffnung des Werkzeugs realisierten Hinterschnitte (Entformung) unter Beanspruchung der Nachgiebigkeit des elastomeren Werkstoffs mindestens bereichsweise zwangsentformt werden.

Im. Gehäuseoberteil sind als Öffnungen ein Lufteinlass und ein Luftauslass zur luftführenden Kopplung mit dem Beatmungsgerät vorgesehen.

Der Anfeuchter wird bevorzugt im Seitenbereich des Beatmungsgerätes angekoppelt, wobei die luftführende Verbindung in Form eines Bypass erfolgt.

Der Lufteinlass und der Luftauslass - die Öffnungen im Oberteil - dienen auch als Einfüllöffnungen für das Wasser.

Im Gehäuseoberteil wird zumindest ein Griff bereitgestellt, Ein Griff dient dazu, das Oberteil vom Unterteil zu lösen.

Im Gehäuseunterteil sind zwei von außen sichtbare Füllstandanzeigen angeordnet, die eine maximale Füllhöhe anzeigen. Eine Füllstandanzeige befindet sich im Bodenbereich des Gehäuseunterteils. Das Gehäuseunterteil ist dazu zumindest teilweise aus einem transparenten Material hergestellt.

Bei Befüllung des Anfeuchters über die Öffnung fließt das Wasser, den vom Mittelteil gebildeten Kanal entlang, in das Unterteil. Dort steigt der Wasserstand im Wasseraufnahmeraum bis zum oberen Rand des Kanals an, dann kann die Luft im Anfeuchter nicht mehr durch den Kanal entweichen und mit weiter steigendem Wasserstand wird die Luft komprimiert wobei der Druck F_{L} der im Luftraum eingeschlossenen Luft auf das im Wasseraufnahmeraum des Anfeuchters befindliche Wasser ansteigt; so entspricht dann der Wasserstand der maximalen Füllhöhe und bei weiterer Befüllung über die Öffnung fließt kein weiteres Wasser in den Wasseraufnahmeraum.

Die gekrümmt angeordneten Luftkanäle des Mittelteiles verhindern zudem ein Zurück- bzw. Auslaufen der Flüssigkeit aus dem Anfeuchter. Die zentrierte Anordnung der Kanäle trägt ebenfalls dazu bei.

Das Mittelteil des Atemluftanfeuchters dient zum Einen der Luftführung durch den Anfeuchter, zum Anderen der Abdichtung zwischen Ober- und Unterteil und auch der Wasserführung beim Befüllvorgang.

Eine einhändige Konnektierung des Anfeuchters an das Beatmungsgerät wird durch korrespondierende Abmessungen im Bereich der Kontaktstelle und einen Schnappmechanismus zur Befestigung erreicht.

Der Anfeuchter wird in einem Seitenbereich an das Beatmungsgerät konnektiert und der Luftweg wird im Bypass durch den Anfeuchter und wieder zurück in das Beatmungsgerät geführt, wobei der Schlauchanschluss am Beatmungsgerät verbleibt.

In den folgenden Zeichnungen sind Ausführungsbeispiele der Erfindung dargestellt. Es zeigen:
- Fig. 1: Aufbau einer Atemgasversorgung ohne Anfeuchter
- Fig. 2: Beatmungsgerät mit abgenommenem Seitenteil
- Fig. 3: Anfeuchter mit seitlicher Aufnahme des Beatmungsgerätes
- Fig. 4: Aufbau eines Beatmungsgerätes mit angeschlossenem. Anfeuchter
- Fig. 5: Anfeuchter
- Fig. 6: Anfeuchter zum Befüllen geschwenkt
- Fig. 7: Explosionszeichnung des Anfeuchters
- Fig. 8: Perspektive Gehäuseunterteil
- Fig. 9: Unteransicht Gehäuseunterteil
- Fig. 10: Mittelteil
- Fig. 10a: Schnitt durch Mittelteil
- Fig. 11: Gehäuseoberteil
- Fig. 12: Schema Anfeuchter, Befüllvorgang

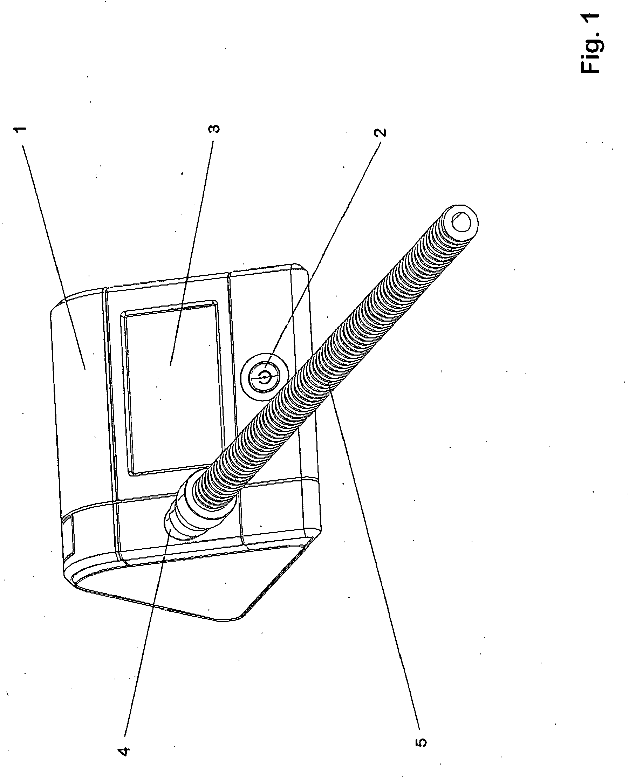

Fig. 1 zeigt den grundsätzlichen Aufbau einer Atemgasversorgung, welche ohne Atemluftanfeuchter ausgeführt ist. Die Atemgasversorgung (1) weist ein Bedienfeld (2) sowie eine Anzeige mit Touchscreen (3) auf, Die Atemgasversorgung ist darüber hinaus im Geräteinnenraum mit einer Fördereinrichtung für das Atemgas in Form einer Atemgaspumpe, einem elektrischen Antrieb, sowie einer Steuerung für den elektrischen Antrieb ausgestattet. Über ein Anschlusselement (4) wird ein Beatmungsschlauch (5) angeschlossen an dessen Ende sich die Atemgasversorgung des Patienten in Form einer Beatmungsmaske oder Beatmungsbrille befindet.

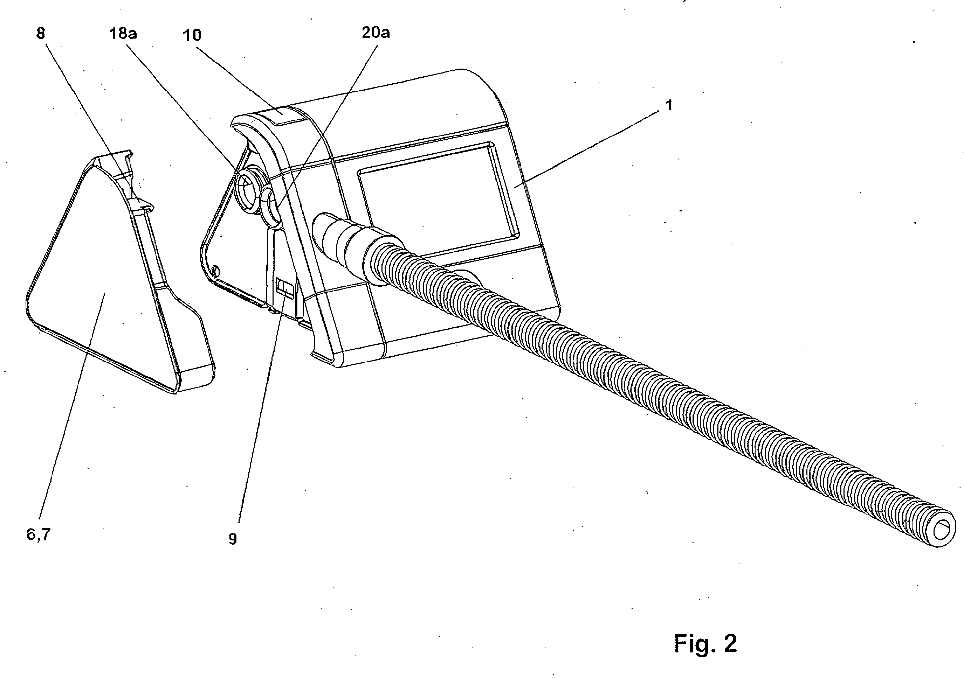

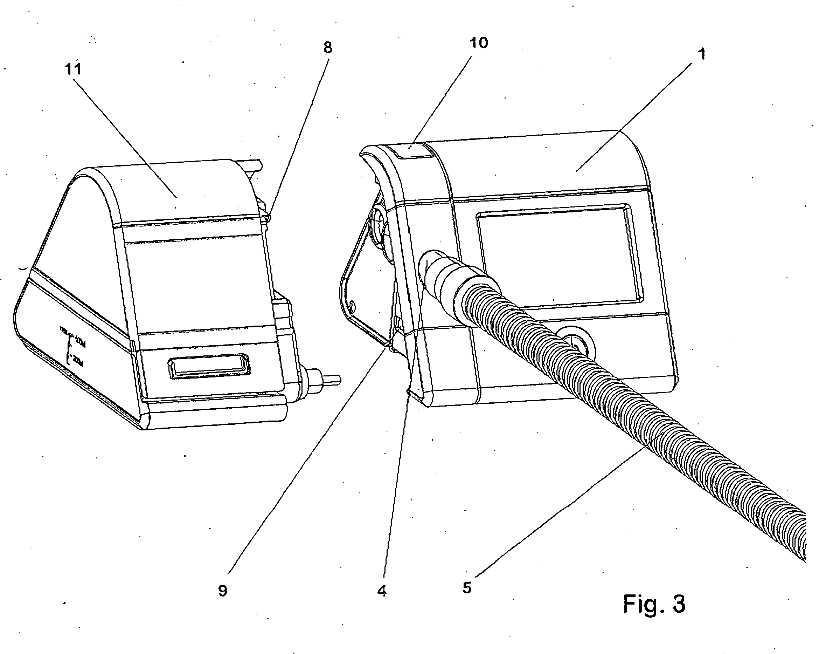

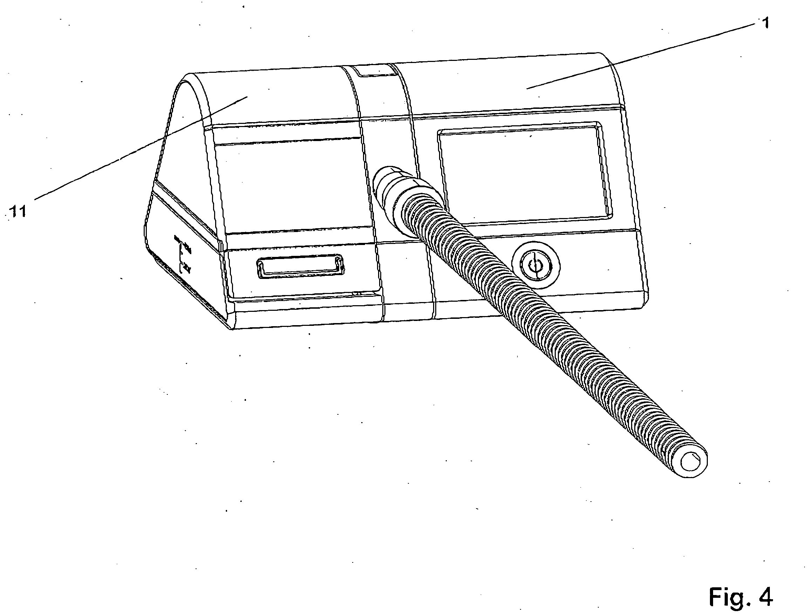

Fig. 2: Um den Atemluftanfeuchter (11) an das Beatmungsgerät (1) zu koppeln wird die Seitenwand (6) des Beatmungsgerätes (1), die einen integrierten Schalldämpfer (7) aufweist, der mit zwei Schnapphaken (8) in Aufnahmen (9) im Beatmungsgerät (1) verrastet, gelöst und gegen das Atemluftanfeuchtermodul (11) ausgetauscht. Das Beatmungsgerät weist im Seitenbereich eine Luftaustritts- (18a) und eine Lufteintrittsöffnung (20a) auf, die entweder im Bypass über den Schalldämpfer verbunden werden oder über den Anfeuchter (11). Hierzu muss die Entriegelung (10) am Beatmungsgerät (1) betätigt werden. Durch Betätigen der Entriegelung (10) werden die Aufnahmen (9), die auf einer Verbindungsschiene miteinander verbunden sind, verschoben und die Verbindung zu den Schnapphaken (8) gelöst. Der Atemluftanfeuchter (11) weist ebenfalls je einen Schnapphaken (8) im Oberteil und Unterteil auf, die in die gleichen Aufnahmen (9) des Beatmungsgerätes (1) greifen. Dieses wird in den Figuren 2 bis 4 dargestellt.

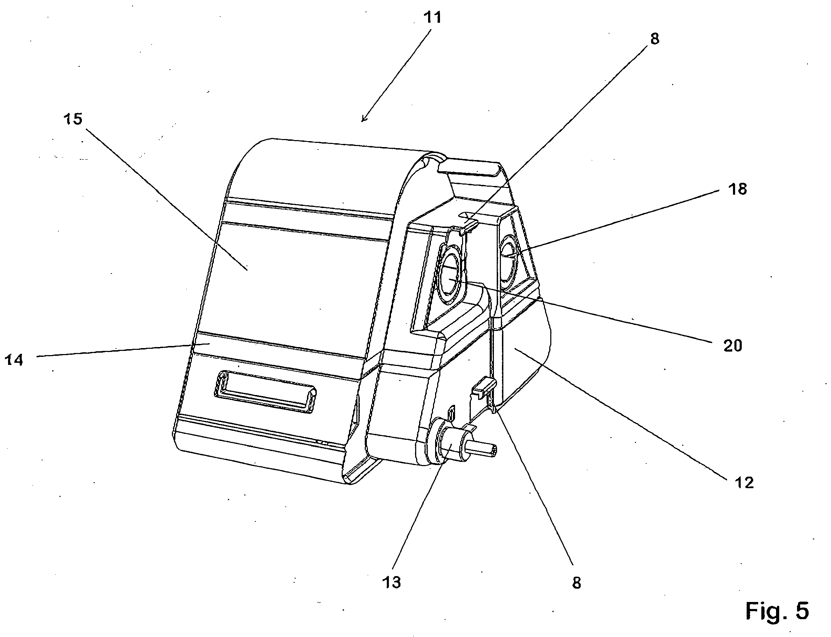

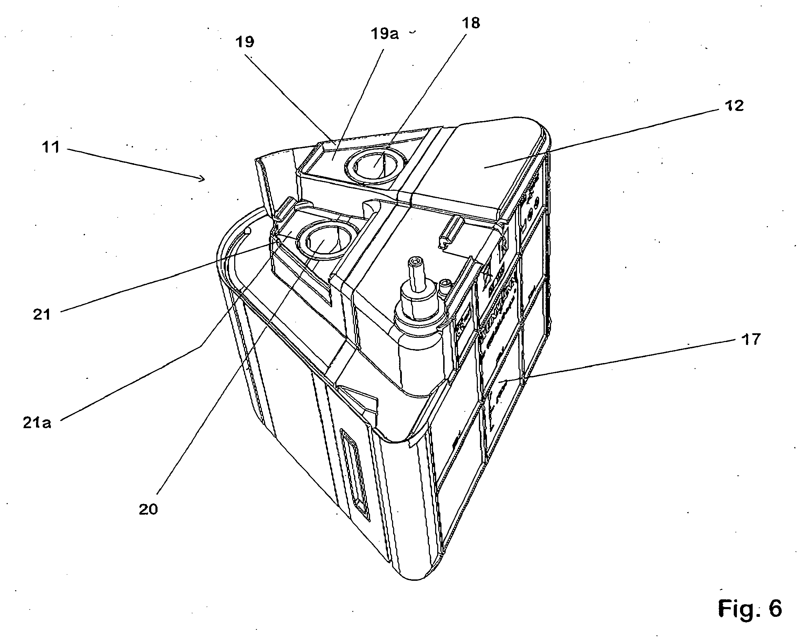

Die Figuren 5 und 6 zeigen den Atemluftanfeuchter (11) im montierten Zustand, welcher aus einem Gehäuseunterteil (12) mit Heizelement (13), einem Mittelteil bevorzugt aus Silikon (14) mit einer Dichtauflage (22a) zum Gehäuseunterteil (12) und einem Gehäuseoberteil (15) besteht.

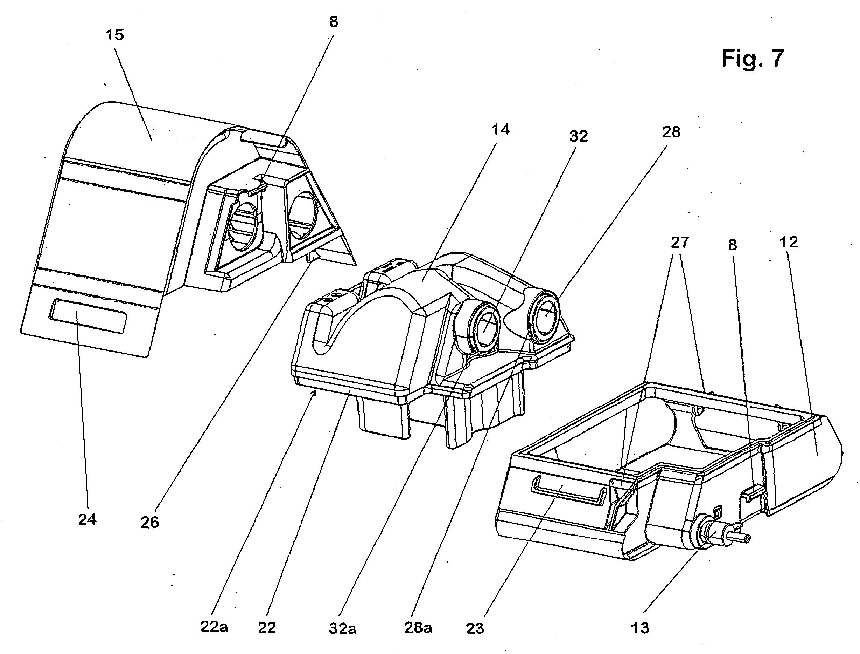

Fig. 7 zeigt in einer Explosionsansicht die Einzelteile, des Anfeuchtermoduls (11). Zur Montage wird das Mittelteil aus Elastomer, bevorzugt Silikon (14), in das Gehäuseoberteil (15) eingesetzt. Das Mittelteil dichtet mit der Dichtfläche (22a) seines Dichtrandes (22) auf dem Unterteil (12) ab, wenn Ober-(15) und Unterteil (12) miteinander verbunden werden. Das Verbinden der Teile erfolgt über Rasthaken (23) am Unterteil (12) und entsprechenden Öffnungen (24) für die Rasthaken (23) im Oberteil. Zum Lösen der Verbindung ist eine Grifffläche (25) im Bereich unter dem Schenkel des Oberteiles (15) vorgesehen. Eine korrespondierende Aussparung (25a) im Unterteil ermöglicht das Greifen, Mit leichter Zugbewegung lösen sich die Rastverbindungen (23,24) aufgrund der Elastizität des verwendeten KunststoffMaterials.

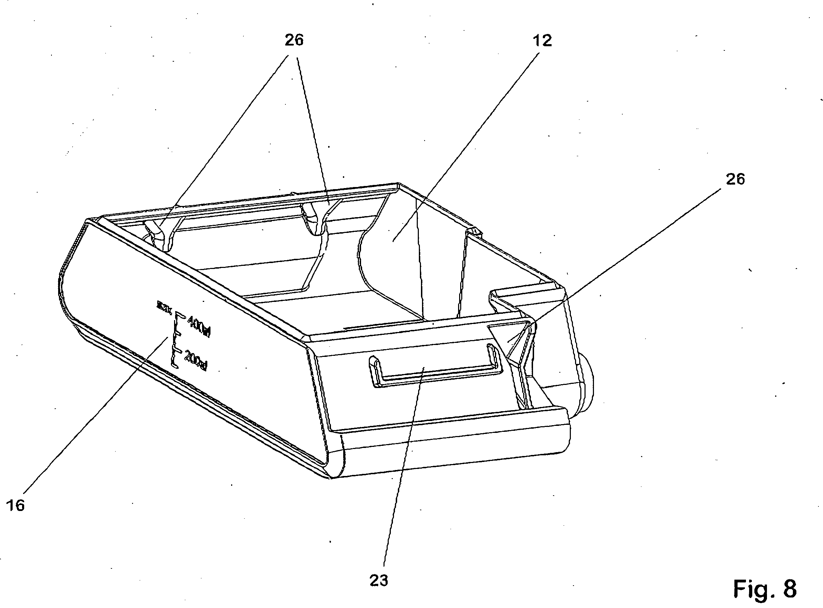

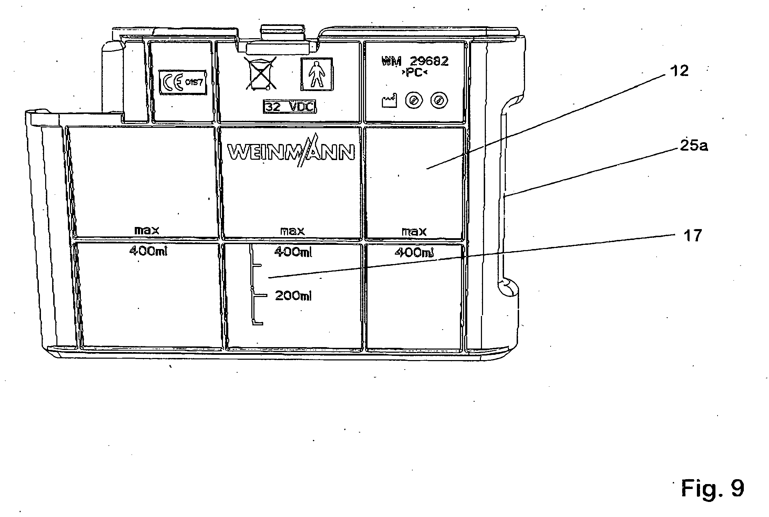

Das Gehäuseunterteil (12) (in den Figuren 8 und 9 dargestellt) weist eine Öffnung für das Heizelement (13) auf, das seitlich in die Öffnung des Gehäuseunterteiles (12) eingeschraubt und mit einem O-Ring abgedichtet wird. Beim Anbau des Anfeuchters (11) an das Beatmungsgerät (1) wird das Heizelement (13) mit der Elektronik des Beatmungsgerätes (1) gekoppelt. Der Anfeuchter wird über das Beatmungsgerät (1) gesteuert.

Der Innenraum des Gehäuseunterteiles (12) dient zur Aufnahme des Wassers, wobei der Wasserstand über zwei Füllstandanzeigen (16 und 17) abgelesen werden kann. Eine Füllstandsanzeige (16) befinden sich im Seitenbereich des Unterteils und dient der Ermittlung des Wasserfüllstandes im Betrieb. Die andere Füllstandsanzeige befindet sich im Bodenbereich des Unterteils und dient der Ermittlung des Wasserfüllstandes (bei einem um 90° gekippten Anfeuchter) bei Befüllung durch die Öffnung (18, 20). Das Anfeuchtermodul (11) kann auf verschiedene Arten mit Wasser befüllt werden. Zum Einen kann er im zerlegten Zustand, also ohne Mittel-und Oberteil, befüllt werden, dafür dient die seitliche Füllstandanzeige (16) im Gehäuseunterteil (12). Zum Anderen kann das Anfeuchtermodul aber auch im zusammengebauten Zustand befüllt werden. Hierzu wird das Anfeuchtermodul (11), wie in Figur 6 dargestellt, geschwenkt und kann über den Lufteinlass (18) oder den Luftauslass (20) befüllt werden. Dafür ist eine zweite Füllstandanzeige (17) im Boden des Gehäuseunterteiles (12) zur Überwachung des Wasserstandes vorgesehen (ebenfalls in Fig.9 dargestellt). Im Bereich der Luft-Einlassöffnung (18) bzw. - Auslassöffnung (20) befindet sich jeweils eine erhöhte Kante (19, 20) die verhindert, dass beim Befüllen des Anfeuchters (11) das Wasser danebenlaufen kann. Die Flächen (19a, 21a) benachbart zu den Öffnungen 18 und 20 sind leicht trichterförmig ausgeführt, um das Wasser in Richtung der Öffnung zu führen. Wird der Anfeuchter (11) wie in Fig. 6 dargestellt mit Wasser befüllt, dient ein Überlaufschutz dazu, dass nicht zu viel Wasser eingefüllt werden kann. Der Überlaufschutz wird durch die Krümmungen der Luftkanäle (29 und 31) des Mittelteiles (14) realisiert. Da aus dem Innenraum keine Luft entweichen kann, wird durch das eingefüllte Wasser die Luft im Innenraum komprimiert und das Wasser steigt in den Luftkanälen (29 und 31) bis zu den Öffnungen (28 und 32) an. Wird der Anfeuchter (11) zurückgeschwenkt, läuft das Wasser in den Innenraum. Die gekrümmt angeordneten Luftkanäle (29 und 31) verhindern zudem ein Zurück- bzw. Auslaufen der Flüssigkeit aus dem Anfeuchter wenn dieser transportiert wird.

Fig. 8 zeigt zusätzlich ein weiteres Detail des Gehäuseunterteiles (12). Es sind Zentrierhilfen (26, 27) in Form von Führungsaufnahmen (26) des Gehäuseunterteiles (15) vorgesehen, in die entsprechenden Führungsstege (27) des Gehäuseoberteiles (15) eingreifen und so einen falschen Zusammenbau der Bauteile verhindern. Die Führungsaufnahmen (26) weisen eine trichterförmige Öffnung, die in einen Schlitz übergeht, auf. Die trichterförmige Öffnung dient zum Einführen des Führungssteges (27) in die Führungsaufnahme (26). Zudem sind die Führungsstege bzw. -aufnahmen asymmetrisch an Gehäuseunterteil (12) und Oberteil (15) angeordnet, was dazu beiträgt, dass es zu keiner falschen Montage führen kann.

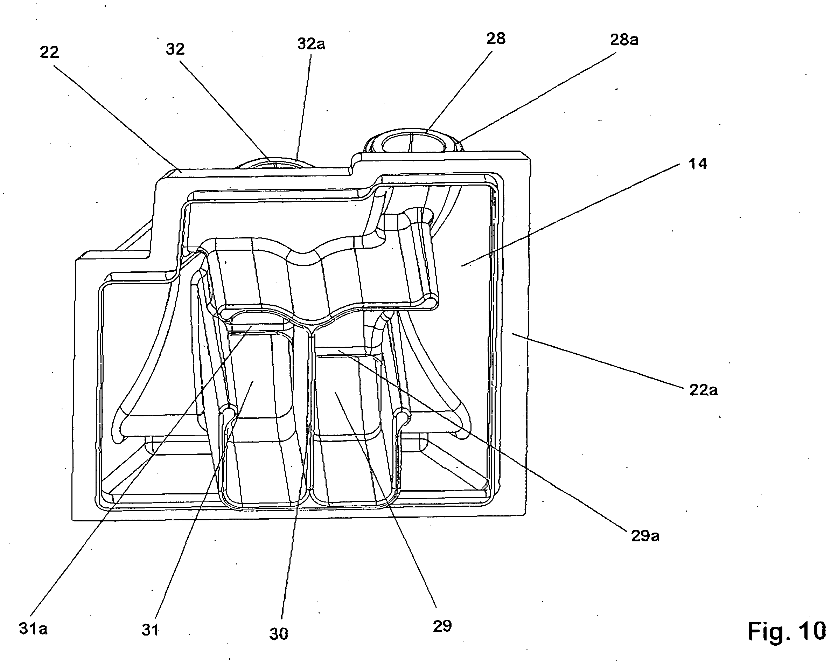

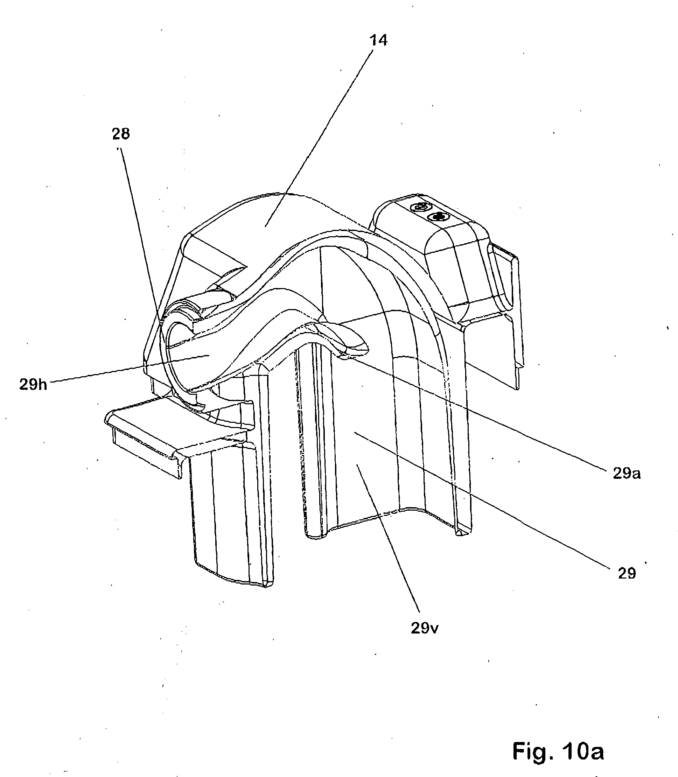

Fig. 10 zeigt das Mittelteil (14) des Atemluftanfeuchters (11). Es dient zum Einen der Luftführung durch den Anfeuchter und zum Anderen der Abdichtung. Durch das gewählte Material, in diesem Fall Silikon oder ein Elastomer welches im 2K-Verfahren hergestellt wurde, kann das Mittelteil (14) beide Anforderungen in Einem erfüllen. Das Mittelteil (14) ist zum Gehäuseoberteil (15) komplett geschlossen und zum Gehäuseunterteil mit dem Wasserreservoir (12) hin innen offen ausgeführt. Das Mittelteil (14) definiert dadurch einen genauen Luftweg durch den Anfeuchter. Der Luftstrom tritt durch die waagerechte Einlassöffnung (28) in einem Winkelbereich von 90° in das Mittelteil (14) ein, wird dann durch den Einlasskanal (29) derart geführt, das er zuerst in einem Winkel von ca. 110 - 120° nach oben und anschließend nach einem Bogen senkrecht nach unten in den Anfeuchterraum im Gehäuseunterteil (12) geleitet wird. Das Mittelteil leitet die Luft so senkrecht auf die Wasserfläche. Dort wird der Luftstrom angefeuchtet und durch den Luftkanal (31) in gleicher Weise zum Luftauslass (32) geführt. Durch das gewählte Material und den Konturverlauf der Kanäle durch das Mittelteil (14) wird der Luftstrom so beruhigt, dass keine zusätzliche Schalldämmung nötig ist. Der Dichtrand (22) des Mittelteiles (14) ist aus einem weicheren Elastomer bevorzugt Silikon ausgeführt und wird beispielsweise im 2K-Verfahrenmit dem Mittelteil (14) verbunden.

Durch den Luftauslass (32) wird der Luftstrom über einen Anschlussstutzen (33) zurück in das Beatmungsgerät (1) geführt und dort durch den Beatmungsschlauch (5) dem Patienten über eine Beatmungsmaske oder -Brille zugeführt. Eine Trennwand (30) zwischen den Luftkanälen (29 und 31), die bis in das eingefüllte Wasser im Gehäuseunterteil (12) reicht, sorgt dafür, dass keine unangefeuchtete Luft in den Luftauslasskanal (31) gelangen kann. Der Dichtrand (28a, 32a) des Mittelteiles (14), welcher benachbart zum Einlass bzw. Auslass (28, 32) angeordnet ist und somit der Abdichtung zum Beatmungsgerät hin dient, ist aus einem weicheren Elastomer bevorzugt Silikon ausgeführt und wird beispielsweise im 2K-Verfahren mit dem Mittelteil (14) verbunden.

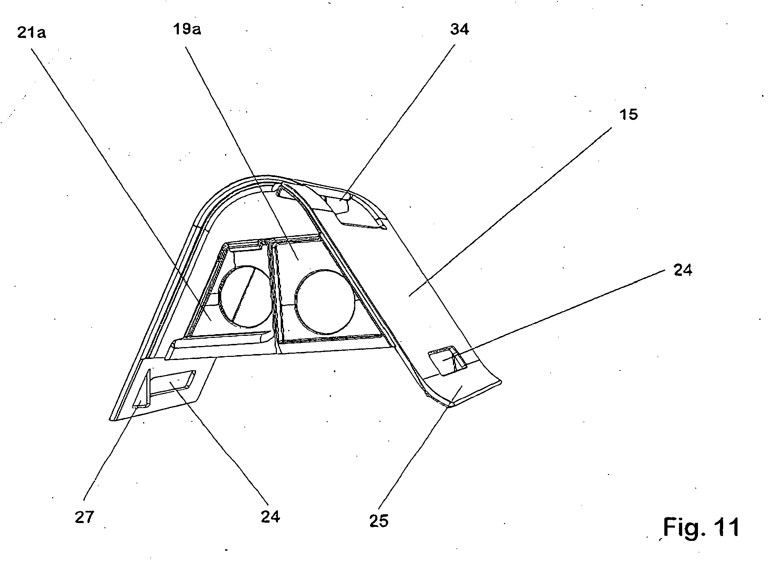

Fig. 11 zeigt das Gehäuseoberteil (15) mit den Öffnungen (24), die beim Zusammenbau des Anfeuchters (11) über die Rasthaken (23) des Gehäuseunterteiles (12) greifen und den zusätzlichen Führungsstegen (27) zur Justierung des Gehäuseoberteiles (12). Das Oberteil weist zumindest einen Griff (25,34) auf. Eine Griffmulde (34) dient zum Tragen des Anfeuchters (11). Die Griffmulde (34) ist identisch zu einer Griffmulde am Beatmungsgerät (1) ausgeführt. Zum Lösen der Verbindung zwischen Ober- und Unterteil ist eine Grifffläche (25) im Bereich des Schenkels des Oberteiles (15) vorgesehen.

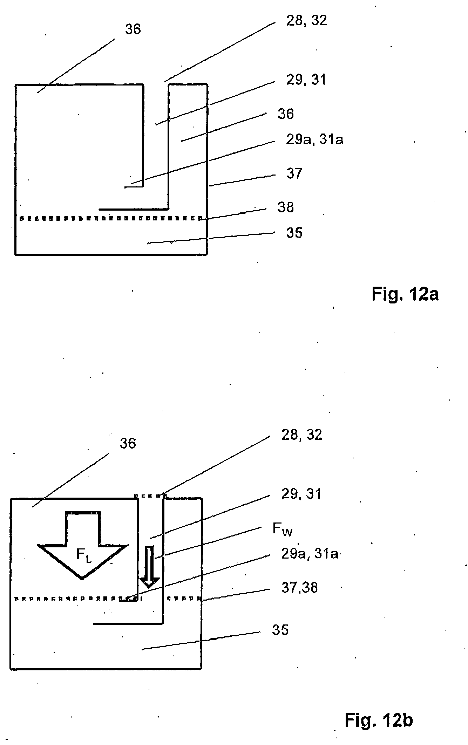

Figur 12 zeigt: Bei dem Befüllen des Anfeuchters (11) mit Wasser über die Öffnung (28 oder 32) steigt die Flüssigkeit im Wasseraufnahmeraum (35) des Anfeuchters langsam an. Das im Wasseraufnahmeraum (35) des Anfeuchters befindliche Volumen an Luft (36) entweicht zunächst sukzessiv durch die Öffnung (28,32). Steigt der Wasserstand (38) bis zum oberen Rand (29a, 31a) des Luftkanals (29,31) an, so kann die Luft nicht mehr entweichen, sie verbleibt im Luftraum (36). Das verbleibende Volumen an Luft wird mit weiter steigendem Wasserstand (38) im Wasseraufnahmeraum (35) des Anfeuchters komprimiert. Dies hat zur Folge, daß der Druck der eingeschlossenen Luft im Luftraum (36) des Anfeuchters auf das im Wasseraufnahmeraum (35) des Anfeuchters befindliche Wasser ansteigt. Ist der Druck (F_{L}) der eingeschlossenen Luft auf das im Wasseraufnahmeraum (35) des Anfeuchters befindliche Wasser, größer als die Kraft (F_{W}) des in dem Kanal (29,31) befindlichen eingefüllten Wassers, so ist die maximale Füllhöhe (37) des Anfeuchters (11) erreicht. Der Wasserstand (38) entspricht dann der maximalen Füllhöhe (37), die vom Anwender auch über die Füllstandanzeige (17) ablesbar ist. Bei weiterer Befüllung über die Öffnung (28, 32) fließt dann kein weiteres Wasser in den Wasseraufnahmeraum (35) der Anfeuchters. Das eingefüllte Wasser steigt dann in beiden Schenkeln des Luftkanals (29,31) bis zur Einfüllöffnung (28,32) empor, ohne die Wassermenge im Anfeuchter weiter zu erhöhen, und läuft über.

### Pos.-Nr.

- 1: Beatmungsgerät
- 2: Bedienfeld
- 3: Anzeige mit Touchscreen
- 4: Anschlusselement für Beatmungsschlauch
- 5: Beatmungsschlauch
- 6: Gehäuse Seitenwand
- 7: Integrierter Schalldämpfer in der Seitenwand (6)
- 8: Schnapphaken
- 9: Aufnahme für Schnapphaken
- 10: Entriegelung
- 11: Atemluftanfeuchter kpl.
- 12: Gehäuseunterteil Anfeuchter
- 13: Heizelement
- 14: Mittelteil
- 15: Gehäuseoberteil Anfeuchter
- 16: Füllstandanzeige im Seitenbereich des Unterteils
- 17: Füllständanzeige im Bodenbereich des Unterteils
- 18: Lufteinlass
- 18a: Luftaustrittsöffnung aus Beatmungsgerät
- 19: Rand um Lufteinlass
- 19a: Fläche um Lufteinlass
- 20: Luftauslass
- 20a: Lufteintrittsöffnung in Beatmungsgerät
- 21: Rand um Luftauslass
- 21a: Fläche um Luftauslass
- 22: Dichtrand an (14)
- 23: Rasthaken in (12)
- 24: Öffnungen in (15)
- 25: Grifffläche am Gehäuseoberteil

- 25a: Aussparung für Griff am Gehäuseunterteil
- 26: Führungsaufnahme im Gehäuseunterteil (12)
- 27: Führungsstege am Gehäuseoberteil (15)
- 28: Lufteinlass im Mittelteil (14)
- 28a: Dichtrand am Einlass (28)
- 29: Luftkanal
- 30: Trennwand
- 31: Luftkanal zum Auslass im Mittelteil (14)
- 32: Luftauslass im Mittelteil (14)
- 32a: Dichtrand am Auslass (32)
- 33: Anschlussstutzen im Beatmungsgerät (1)
- 34: Griffmulde im Gehäuseoberteil (15)
- 35: Wasseraufnahmeraum
- 36: Luftraum
- 37: Füllhöhe
- 38: Wasserstand

## Patentansprüche

1. Atemluftanfeuchter zur Verwendung mit einem Beatmungsgerät bestehend aus einem Gehäuseunterteil (12) mit Heizelement (13), einem Gehäuseoberteil (15) und einem elastomeren Mittelteil (14), wobei das Mittelteil (14) eine Dichtauflage (22) zum Gehäuseunterteil (12) aufweist, das Mittelteil (14) einstückig ausgebildet ist und Elemente zur Strömungsführung und/oder Strömungskanäle (28, 29, 30, 31, 32) und Dichtelemente (22, 28a, 32a) zur pneumatischen Abdichtung aufweist, **dadurch gekennzeichnet, dass** das Mittelteil (14) aus mindestens 2 Elastomeren verschiedener Härtegrade (Shorehärten) besteht und die strömungsführenden Elemente des elastomeren Mittelteils (14) eine höhere Shorehärte als die Dichtelemente (22, 28a, 32a) aufweisen, wobei die strömungsführenden Elemente des elastomeren Mittelteils eine Shorehörte zwischen 40 und 100 Shore A und die Dichtelemente des elastomeren Mittelteils eine Shorehärte zwischen 5 und 70 Shore A aufweisen

2. Atemluftanfeuchter nach Anspruch 1 **dadurch gekennzeichnet, dass** der Dichtrand (22) des Mittelteiles (14) aus einem weicheren Elastomer ausgeführt ist, welches im 2K-Verfahren mit dem Mittelteil (14) verbunden ist.

3. Atemluftanfeuchter nach einem der vorhergehenden Ansprüche **dadurch gekennzeichnet, dass** die Elemente zur Strömungsführung und/oder Strömungskanäle (28, 29, 30, 31, 32) mindestens eine Umlenkung der Luft von horizontal nach vertikal und/oder eine Umlenkung von vertikal in eine horizontale Strömungsrichtung zum Anfeuchterausgang hin bewirken.

4. Atemluftanfeuchter nach zumindest einem der vorhergehenden Ansprüche **dadurch gekennzeichnet, dass** der Luftstrom durch die waagerechte Einlassöffnung (28) in einem Winkelbereich von 90° in das Mittelteil (14) eintritt und dann durch den Einlasskanal (29) derart geführt wird, das er zuerst in einem Winkel von ca. 110 - 120° nach oben und anschließend nach einem Bogen senkrecht nach unten in den Anfeuchterraum im Gehäuseunterteil (12) geleitet wird.

5. Atemluftanfeuchter nach zumindest einem der vorhergehenden Ansprüche **dadurch gekennzeichnet, dass** die Elemente zur Strömungsführung und/oder Strömungskanäle (28, 29, 30, 31, 32) eine Aufteilung der Luftströmung in mindestens zwei Teilströme oberhalb der Wasseroberfläche bewirken und die Teilströme vor dem Luftauslass (20) wieder vereinigt werden.

6. Atemluftanfeuchter nach zumindest einem der vorhergehenden Ansprüche **dadurch gekennzeichnet, dass** eine Trennwand (30) zwischen den Luftkanälen (29 und 31) angeordnet ist, die bis in das eingefüllte Wasser im Gehäuseunterteil (12) reicht, und die dafür sorgt, dass keine unangefeuchtete Luft in den Luftauslasskanal (31) gelangen kann.

7. Atemluftanfeuchter nach zumindest einem der vorhergehenden Ansprüche **dadurch gekennzeichnet, dass** das elastomere Mittelteil (14) rohrförmige Anschlussstellen bereitstellt die mit Öffnungen (18, 20) im Gehäuseoberteil gemeinsam jeweils einen Lufteinlass (18) und einen Luftauslass (20) bilden und zur luftführenden Kopplung mit dem Beatmungsgerät vorgesehen sind, wobei das Mittelteil im Kopplungsbereich zum Beatmungsgerät Dichtelemente (28a, 32a) zur Abdichtung zum Beatmungsgerät hin aufweist.

8. Atemluftanfeuchter nach zumindest einem der vorhergehenden Ansprüche **dadurch gekennzeichnet, dass** der Dichtrand (28a, 32a) des Mittelteiles (14), welcher benachbart zum Einlass bzw. Auslass (28, 32) angeordnet ist und somit der Abdichtung zum Beatmungsgerät hin dient, aus einem weicheren Elastomer ausgeführt ist und im 2K-Verfahren mit dem Mittelteil (14) verbunden ist.

9. Atemluftanfeuchter nach zumindest einem der vorhergehenden Ansprüche **dadurch gekennzeichnet, dass** der Lufteinlass (18) und/oder der Luftauslass (20) auch als Einfüllöffnungen für das Wasser dienen wobei der Lufteinlass und Luftauslass benachbart angeordnet sind und eine Befüllung mit Wasser (ohne Fehlbedienungsrisiko) wahlweise durch eine der beiden Öffnungen vorgenommen werden kann.

10. Atemluftanfeuchter nach zumindest einem der vorhergehenden Ansprüche **dadurch gekennzeichnet, dass** Luftkanäle im Mittelteil beim Übergang zwischen vertikalen (29v, 31v) und horizontalen Abschnitten (29h, 31h) geometrische Überhöhungen (29a, 31a) aufweisen.

11. Atemluftanfeuchter nach zumindest einem der vorhergehenden Ansprüche **dadurch gekennzeichnet, dass** das Gehäuseoberteil (15) im Wesentlichen der mechanischen Abstützung des elastomeren Mittelteils sowie der mechanischen Verriegelung mit dem Unterteil dient, wobei das Mittelteil zwischen Oberteil und Unterteil eingespannt wird und die Dicht(-auflage)elemente des elastomeren Mittelteils (14) den von Mittelteil (14) und Gehäuseunterteil (12) gemeinsam gebildeten Wasseraufnahmeraum (35) gegenüber der Umgebung abdichten.

12. Atemluftanfeuchter nach zumindest einem der vorhergehenden Ansprüche **dadurch gekennzeichnet, dass** im Gehäuseunterteil Führungsaufnahmen (26) zur Justierung des Gehäuseoberteiles (15) angeordnet sind, in die entsprechende Führungsstege (27) des Gehäuseoberteiles (15) greifen, wobei die Führungsstege und - aufnahmen (26, 27) asymmetrisch an Gehäuseunterteil (12) und Oberteil (15) angeordnet sind um eine definierte Orientierung bei der Montage vorzugeben.

13. Atemluftanfeuchter nach zumindest einem der vorhergehenden Ansprüche **dadurch gekennzeichnet, dass** die Führungsaufnahmen (26) jeweils eine trichterförmige Öffnung aufweisen die zum Einführen der Führungsstege (27) in die Führungsaufnahmen (26) dienen.

14. Atemluftanfeuchter nach zumindest einem der vorhergehenden Ansprüche **dadurch gekennzeichnet, dass** im Gehäuseunterteil zwei von außen sichtbare Füllstandanzeigen (16, 17) angeordnet sind.

15. Atemluftanfeuchter nach zumindest einem der vorhergehenden Ansprüche **dadurch gekennzeichnet, dass** der Atemluftanfeuchter lediglich aus vier Bauteilen, nämlich einem Gehäuseunterteil (12) mit Heizelement (13), einem Gehäuseoberteil (15) und einem elastomeren Mittelteil besteht.

## Claims

1. A respiratory air humidifier for use with a ventilator consisting of a lower housing part (12) with a heating element (13), an upper housing part (15) and an elastomer central part (14), wherein the central part (14) has a sealing layer (22) to the lower housing part (12), the central part (14) is designed as a single piece and has elements for flow guiding and/or flow channels (28, 29, 30, 31, 32) and sealing elements (22, 28a, 32a) for pneumatic sealing, **characterized in that** the central part (14) consists of at least two elastomers of various degrees of hardness (Shore hardnesses) and the flow-guiding elements of the elastomer central part (14) have a higher Shore hardness than the sealing elements (22, 28a, 32a), wherein the flow-guiding elements of the elastomer central part have a Shore hardness between 40 and 100 Shore A and the sealing elements of the elastomer central part have a Shore hardness between 5 and 70 Shore A.

2. The respiratory air humidifier according to claim 1, **characterized in that** the sealing edge (22) of the central part (14) is implemented from a softer elastomer which is connected to the central part (14) in a bi-component method.

3. The respiratory air humidifier according to one of the preceding claims, **characterized in that** the elements for flow guiding and/or flow channels (28, 29, 30, 31, 32) cause at least one redirection of the air from horizontal to vertical and/or one redirection from vertical to a horizontal flow direction toward the humidifier output.

4. The respiratory air humidifier according to at least one of the preceding claims, **characterized in that** the air flow enters into the central part (14) through the horizontal inlet opening (28) at an angle range of 90° and is then guided through the inlet channel (29) such that it is first conducted upwards at an angle of approx. 110 - 120° and then vertically downwards after a curve into the humidifier space in the lower housing part (12).

5. The respiratory air humidifier according to at least one of the preceding claims, **characterized in that** the elements for flow guiding and/or flow channels (28, 29, 30, 31, 32) cause the air flow to split into at least two partial streams above the surface of the water and the partial streams are joined again before the air outlet (20).

6. The respiratory air humidifier according to at least one of the preceding claims, **characterized in that** a dividing wall (30) that reaches into the water filled in the lower housing part (12) and that ensures that non-humidified air cannot enter the air outlet channel (31) is arranged between the air channels (29 and 31).

7. The respiratory air humidifier according to at least one of the preceding claims, **characterized in that** the elastomer central part (14) provides tubular connection points that together each form an air inlet (18) and an air outlet (20) with openings (18, 20) in the upper housing part and that are provided for air-guiding coupling with the ventilator, wherein the central part has sealing elements (28a, 32a) for sealing toward the ventilator in the coupling area to the ventilator.

8. The respiratory air humidifier according to at least one of the preceding claims, **characterized in that** the sealing edge (28a, 32a) of the central part (14), which is arranged adjacent to the inlet or respectively outlet (28, 32) and accordingly serves to seal toward the ventilator, is implemented from a softer elastomer and is connected to the central part (14) in the bi-component method.

9. The respiratory air humidifier according to at least one of the preceding claims, **characterized in that** the air inlet (18) and/or the air outlet (20) also serve as filling openings for the water, wherein the air inlet and air outlet are arranged adjacent and water can be filled (without the risk of operating error) optionally through one of the two openings.

10. The respiratory air humidifier according to at least one of the preceding claims, **characterized in that** air channels in the central part have geometric elevations (29a, 31a) at the transition between vertical (29v, 31v) and horizontal sections (29h, 31h).

11. The respiratory air humidifier according to at least one of the preceding claims, **characterized in that** the upper housing part (15) serves substantially to mechanically support the elastomer central part as well as to mechanically latch with the lower part, wherein the central part is clamped between the upper part and the lower part and the sealing (layer) elements of the elastomer central part (14) seal off the water receiving space (35) formed by the central part (14) and the lower housing part (12) from the environment.

12. The respiratory air humidifier according to at least one of the preceding claims, **characterized in that** guide receivers (26) for adjusting the upper housing part (15) are arranged in the lower housing part with which corresponding guide bars (27) of the upper housing part (15) engage, wherein the guide bars and receivers (26, 27) are arranged asymmetrically on the lower housing part (12) and the upper part (15) in order to give a defined orientation during assembly.

13. The respiratory air humidifier according to at least one of the preceding claims, **characterized in that** the guide receivers (26) each have a funnel-shaped opening that serve to introduce the guide bars (27) into the guide receivers (26).

14. The respiratory air humidifier according to at least one of the preceding claims, **characterized in that** two fill-level indicators (16, 17) that are visible from the outside are arranged in the lower housing part.

15. The respiratory air humidifier according to at least one of the preceding claims, **characterized in that** the respiratory air humidifier consists of only four components, namely a lower housing part (12) with a heating element (13), an upper housing part (15) and an elastomer central part.

## Revendications

1. Humidificateur d'air respiratoire destiné à être utilisé avec un appareil respiratoire constitué d'une partie inférieure de boîtier (12) comprenant un élément chauffant (13), d'une partie supérieure de boîtier (15) et d'une partie intermédiaire élastomère (14), dans lequel la partie intermédiaire (14) présente un revêtement d'étanchéité (22) par rapport à la partie inférieure de boîtier (12), la partie intermédiaire (14) est constituée d'une seule pièce et comporte des éléments destinés à guider l'écoulement et/ou des canaux d'écoulement (28, 29, 30, 31, 32) et des éléments d'étanchéité (22, 28a, 32a) assurant l'étanchéité pneumatique, **caractérisé en ce que** la partie intermédiaire (14) est constituée d'au moins deux élastomères avec différents degrés de dureté (dureté Shore), et les éléments de guidage d'écoulement de la partie intermédiaire élastomère (14) présentent une dureté Shore supérieure à celle des éléments d'étanchéité (22, 28a, 32a), les éléments de guidage d'écoulement de la partie intermédiaire élastomère présentant une dureté Shore entre 40 et 100 Shore A et les éléments d'étanchéité de la partie intermédiaire élastomère présentant une dureté Shore entre 5 et 70 Shore A.

2. Humidificateur d'air respiratoire selon la revendication 1, **caractérisé en ce que** le bord d'étanchéité (22) de la partie intermédiaire (14) est conçu à partir d'un élastomère plus souple, lequel est relié à la partie intermédiaire (14) à l'aide d'un procédé 2K.

3. Humidificateur d'air respiratoire selon l'une des revendications précédentes, **caractérisé en ce que** les éléments destinés à guider l'écoulement et/ou les - canaux d'écoulement (28, 29, 30, 31, 32) produisent au moins une déviation de l'air de l'horizontale à la verticale et/ou une déviation de la verticale vers une direction d'écoulement horizontale vers la sortie de l'humidificateur.

4. Humidificateur d'air respiratoire selon l'une au moins des revendications précédentes, **caractérisé en ce que** le flux d'air entre dans la partie intermédiaire (14) sous une plage angulaire de 90° par l'orifice d'admission horizontal (28), pour être ensuite transporté à travers le canal d'admission (29) de manière à être guidé tout d'abord sous un angle d'environ 110-120° vers le haut, puis verticalement vers le bas après une courbe, dans l'espace d'humidificateur dans la partie inférieure de boîtier (12).

5. Humidificateur d'air respiratoire selon l'une au moins des revendications précédentes, **caractérisé en ce que** les éléments destinés à guider l'écoulement et/ou les canaux d'écoulement (28, 29, 30, 31, 32) produisent une division de l'écoulement d'air en au moins deux flux partiels au-dessus de la surface de l'eau, et **en ce que** les flux partiels sont de nouveau réunis en amont de la sortie d'air (20).

6. Humidificateur d'air respiratoire selon l'une au moins des revendications précédentes, **caractérisé en ce qu'**une cloison de séparation (30) est disposée entre les canaux d'air (29 et 31), laquelle s'étend jusque dans l'eau introduite dans la partie inférieure de boîtier (12), et laquelle est utilisée pour empêcher que de l'air non humidifié-ne parvienne dans le canal de sortie d'air (31).

7. Humidificateur d'air respiratoire selon l'une au moins des revendications précédentes, **caractérisé en ce que** la partie intermédiaire élastomère (14) met à disposition des zones de raccordement tubulaires, lesquelles forment ensemble respectivement une entrée d'air (18) et une sortie d'air (20) avec des orifices (18, 20) dans la partie supérieure de boîtier et sont destinées à l'accouplement avec l'appareil respiratoire de manière à guider l'air, la partie intermédiaire présentant des éléments d'étanchéité (28a, 32a) assurant l'étanchéité par rapport à l'appareil respiratoire dans la région d'accouplement vers l'appareil respiratoire.

8. Humidificateur d'air respiratoire selon l'une au moins des revendications précédentes, **caractérisé en ce que** le bord d'étanchéité (28a, 32a) de la partie intermédiaire (14), lequel est disposé à côté de l'entrée ou de la sortie (28, 32) et assure donc l'étanchéité par rapport à l'appareil respiratoire, est constitué d'un élastomère plus souple et relié à la partie intermédiaire (14) à l'aide d'un procédé 2K.

9. Humidificateur d'air respiratoire selon l'une au moins des revendications précédentes, **caractérisé en ce que** l'entrée d'air (18) et/ou la sortie d'air (20) servent/sert également d'orifice(s) de remplissage pour l'eau, l'entrée d'air et la sortie d'air étant disposées l'une à côté de l'autre et l'eau pouvant être introduite au choix par l'un des deux orifices (sans risque de mauvaise manipulation).

10. Humidificateur d'air respiratoire selon l'une au moins des revendications précédentes, **caractérisé en ce que** des canaux d'air présentent des surélévations géométriques (29a, 31a) dans la partie intermédiaire au niveau de l'interface entre des sections verticales (29v, 31v) et horizontales (29h, 31h).

11. Humidificateur d'air respiratoire selon l'une au moins des revendications précédentes, **caractérisé en ce que** la partie supérieure de boîtier (15) sert essentiellement pour le support mécanique de la partie intermédiaire élastomère ainsi que pour le verrouillage mécanique avec la partie inférieure, la partie intermédiaire étant serrée entre la partie supérieure et la partie inférieure et les éléments (de revêtement) d'étanchéité de la partie intermédiaire élastomère (14) assurant l'étanchéité de l'espace de réception d'eau (35) formé conjointement par la partie intermédiaire (14) et la partie inférieure de boîtier (12) par rapport à l'environnement.

12. Humidificateur d'air respiratoire selon l'une au moins des revendications précédentes, **caractérisé en ce que** des logements de guidage (26) destinés à ajuster la partie supérieure de boîtier (15), dans lesquels s'engagent des nervures de guidage (27) correspondantes de la partie supérieure de boîtier (15), sont disposés dans la partie inférieure de boîtier, les nervures et les logements de guidage (26, 27) étant disposés de façon asymétrique sur la partie inférieure de boîtier (12) et la partie supérieure (15), pour donner une orientation définie lors du montage.

13. Humidificateur d'air respiratoire selon l'une au moins des revendications précédentes, **caractérisé en ce que** les logements de guidage (26) présentent respectivement un orifice conique servant à l'introduction des nervures de guidage (27) dans les logements de guidage (26).

14. Humidificateur d'air respiratoire selon l'une au moins des revendications précédentes, **caractérisé en ce que** deux indicateurs de niveau de remplissage (16, 17) visibles de l'extérieur sont disposés dans la partie inférieure de boîtier.

15. Humidificateur d'air respiratoire selon l'une au moins des revendications précédentes, **caractérisé en ce que** l'humidificateur d'air respiratoire est constitué de seulement quatre pièces, notamment une partie inférieure de boîtier (12) comprenant un élément chauffant (13), une partie supérieure de boîtier (15) et une partie intermédiaire élastomère.
